(19) Europäisches Patentamt
European Patent Office
Office européen des brevets

(11) Publication number: **0 417 033 A2**

# (12) EUROPEAN PATENT APPLICATION

(21) Application number: **90810553.9**

(22) Date of filing: **18.07.90**

(51) Int. Cl.$^5$: **C12P 19/40**, A01N 43/90, C07H 19/16

The applicant has filed a statement in accordance with Rule 28 (4) EPC (issue of a sample only to an expert). Accession number(s) of the deposit(s): DSM 4757.

The title of the invention has been amended (Guidelines for Examination in the EPO, A-III, 7.3).

(30) Priority: **20.07.89 GB 8916601**

(43) Date of publication of application: **13.03.91 Bulletin 91/11**

(84) Designated Contracting States: **AT BE CH DE DK ES FR GB GR IT LI LU NL SE**

(71) Applicant: **SANDOZ LTD.**
**Lichtstrasse 35**
**CH-4002 Basel(CH)**
(84) **BE CH DK ES FR GB GR IT LI LU NL SE**

Applicant: **SANDOZ-PATENT-GMBH**
**Humboldtstrasse 3**
**W-7850 Lörrach(DE)**
(84) **DE**

Applicant: **SANDOZ-ERFINDUNGEN Verwaltungsgesellschaft m.b.H.**
**Brunner Strasse 59**
**A-1235 Wien(AT)**
(84) **AT**

(72) Inventor: **Le-Van, Ngo**
**Rue du Quai 10**
**F-68220 Neuwiller(FR)**
Inventor: **Sanglier, Jean-Jacques**
**Rebgässli 10**
**CH-4123 Allschwil(CH)**

## (54) Production of dehydrosine fungin by microorganisms and agricultural use.

(57) This application relates the use of dehydrosinefungin as a pesticide, a method of producing dehydrosinefungin, and to a microorganism which produces dehydrosinefungin.

EP 0 417 033 A2

## IMPROVEMENTS IN OR RELATING TO ORGANIC COMPOUNDS

This application relates the use of dehydrosinefungin as a pesticide, a method of producing dehydrosinefungin, and to a microorganism which produces dehydrosinefungin.

Dehydrosinefungin is a known compound, and has the structural formula

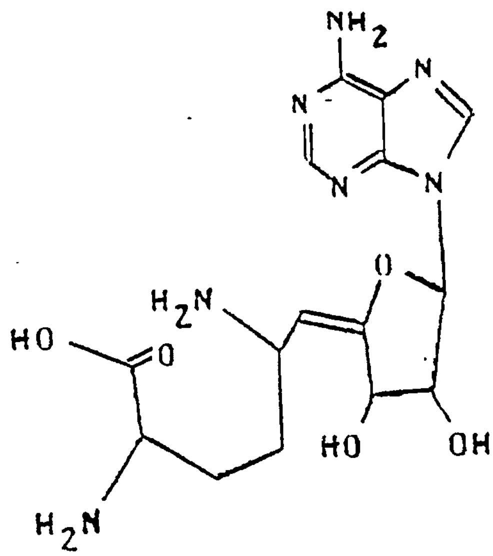

USP 4,087,603 issued May 2, 1978, discloses that minor quantities of dehydrosinefungin are produced along with sinefungin by culturing Streptomyces griseolus NRRL 3739. However, this patent provides no physical or biological data for dehydrosinefungin.

It has now been found that dehydrosinefungin is particularly useful for combatting acari, particularly two-spotted spider mite Tetranychus urticae . This application therefore provides a method of combatting acari comprising applying to the acari or their locus an acaricidally effective amount of dehydrosinefungin.

It has now also been found that dehydrosinefungin demonstrates a high level of herbicidal activity against weeds, particularly the broad-leaves Amaranthus , Sinapis , Solanum , Senecio , Sunflower and Xanthium . This application therefore provides a method of combatting weeds comprising applying to the weeds or their locus a herbicidally effective amount of dehydrosinefungin.

It has now also been found that dehydrosinefungin demonstrates a high level of fungicidal activity against phytopathogenic fungi, particularly Botrytis cinerea , Erysiphe , Sphaerotheca , and Uromyces . This application therefore provides a method of combatting plant pathogens comprising applying to the plant pathogens or their habitat a fungicidally effective amount of dehydrosinefungin.

The appropriate application rates can be determined by routine experiments by those skilled in the art, or by comparing the activity of dehydrosinefungin with standards for which the application rate is known. In general, satisfactory control of acari is obtained when employing dehydrosinefungin at a rate of from about 2 g active ingredient (a.i.)/ha to 100 g a.i./ha of acari-infested habitat. Satisfactory control of weeds is obtained when employing dehydrosinefungin at a rate of from about 100 g a.i./ha to 1 kg a.i./ha of weed-infested habitat. Satisfactory control of plant pathogens is obtained when employing dehydrosinefungin at a rate of from about 2 g/ha to 500 g/ha, preferably from about 100 g/ha to 400 g/ha, of plant pathogen-infested habitat.

Dehydrosinefungin can conveniently be employed in an agricultural compositions in association with agriculturally acceptable carriers for application to the weed, acari, or their loci. Such compositions also form part of the present invention.

Methods of preparing suitable compositions which can be used with dehydrosinefungin are described in the literature along with suitable liquid and solid carriers. The optimum usage of a compound of the present invention is readily determinable by one of ordinary skill in the art using routine testing such as greenhouse testing and small plot testing.

Suitable compositions contain from 0.01 to 99 % by weight of active ingredient, from 0 to 20 % of

surfactant and from 1 to 99.99 % of solid or liquid diluent(s). Higher ratios of surfactant to active ingredient are sometimes desirable and are achieved by incorporation into the formulation or by tank mixing. Application forms of a composition generally contain between 0.01 and 25 % by weight of active ingredient. Lower or higher levels of active ingredient can, of course, be present depending on the intended use, the physical properties of the compound and the mode of application. Concentrate forms of a composition intended to be diluted before use generally contain between 2 and 90 %, preferably between 5 and 85 % by weight of active ingredient.

Useful compositions containing dehydrosinefungin wettable powders, emulsifiable concentrates, and the like. They are obtained by conventional manner, e.g. by mixing a compound of formula 1 with the diluent(s) and optionally with other ingredients.

**Example A**

Preparation of a wettable powder

25 parts of dehydrosinefungin are mixed and milled with 25 parts of synthetic fine silica, 2 parts of sodium lauryl sulphate, 3 parts of sodium ligninsulphonate and 45 parts of finely divided kaolin until the mean particle size is about 5 microns. The resulting wettable powder is diluted with water before use to a spray liquor with the desired concentration.

**Example B**

Preparation of emulsifiable concentrate (EC)

13.37 parts of dehydrosinefungin are mixed in a beaker with 7.04 parts of Toximul 360A® (a mixture of anionic and non-ionic surfactants containng largely anionic surfactants), 23.79 parts of dimethylformamide and 55.8 parts of Tenneco 500-100® (predominantly a mixture of alkylated aromatics such as xylene and ethylbenzene) until solution is effected. The resulting EC is diluted with water for use.

Alternatively, dehydrosinefungin may be used in micro-encapsulated form.

Agriculturally acceptable additives may be employed in the compositions to improve the performance of the active ingredient and to reduce foaming, caking and corrosion, for example.

"Surfactant", as used herein means an agriculturally acceptable material which imparts emulsifiability, spreading, wetting, dispersibility or other surface-modifying properties. Examples of surfactants are sodium lignin sulfonate and lauryl sulfate. "Diluent" as used herein means a liquid or solid agriculturally material sued to dilute a concentrated material to a usable or desirable strength. For dusts or granules it can be e.g. talc, kaolin or diatomaceous earth, for liquid concentrate forms for example a hydrocarbon such as xylene or an alcohol such as isopropanol, and for liquid application forms e.g. water or diesel oil.

The compositions of this invention can also comprise other compounds having biological activity, e.g. compounds having antidotal, fungicidal, insecticidal or insect attractant activity.

A further aspect of this application relates to a microorganism capable of producing dehydrosinefungin in a quantity greater than 10 mg/l of culture as well as to a method of producing dehydrosinefungin by cultivating this microorganism.

Heretofore, dehydrosinefungin has only been produced as a minor metabolite by culturing Streptomyces griseolus NRRL 3739. It has now been found that dehydrosinefungin can be produced in a quantity greater than 10 mg/l of culture by culturing the microorganism Micromonospora sp. A87-16806 under aerobic conditions in a culture medium containing assimilable sources of carbon, nitrogen and mineral salts. Micromonospora sp. A87-16806 was deposited with the Deutsche Sammlung von Mikroorganismen und Zellkulturen GmbH, Mascheroder Weg 1b, D-3300 Braunschweig, Federal Republic of Germany on 27 July 1988 under the terms of the Budapest Treaty, and has been assigned the reference number DSM 4757. This application therefore provides a method of producing dehydrosinefungin which comprises cultivating a microorganism capable of producing dehydrosinefungin in a quantity greater than 10 mg/l of culture under aerobic conditions in a culture medium containing assimilable sources of carbon, nitrogen and mineral salts.

Micromonospora sp. A87-16806 was isolated from a forest soil sample (pH 5.4) obtained from Bangriposi, India. The organism has been classified as a new strain of the genus Micromonospora

(Bergeyl's Manual, 8th Ed., 1974).

Micromonospora sp. A87-16806 grows on various organic and inorganic media, and forms mostly an orange substrate mycelium, which, after spore formation, appears black, with a weak whitish-grey aerial mycelium. The primary substrate mycelium grows as hyphae. On the face of the substrate forms individual black spores in compact clusters. These spores are separate spheres, smooth to bumpy, under 1 micron in diameter. The mass of spores is black. The aerial mycelium is often not well formed, often only at the edge of the colonies, and is mostly grey. The cell walls hold meso- and hydroxy-diaminopimelic acid. Xylose and arabinose are present in the cell hydrolysate. In the fatty-acid spectrum one finds tuberculostearic acid, iso- and anteiso- branched straight chain acids.

The ability of Micromonospora sp. A87-16806 to grow on usual biological media, its carbon assimilation, and its physiological characteristica are presented in the following table.

| Culture medium | Culture characteristics |
|---|---|
| yeast extract/<br>malt extract agar | growth: very good<br>substrate mycelium: orange, then greenish black<br>aerial mycelium: medium growth, grey<br>soluble pigments: none |
| oatmeal | growth: weak<br>substrate mycelium: orange<br>aerial mycelium: none<br>soluble pigments: none |
| Glucose-asparagine agar | growth: medium<br>substrate mycelium: greenish-grey<br>aerial mycelium: none<br>soluble pigments: none |
| yeast extract-starch agar | growth: good<br>substrate mycelium: orange, then greenish-black<br>aerial mycelium: in some places, greyish<br>soluble pigments: none |

| Carbon assimiliation | Carbon source |
|---|---|
| positive | glucose, saccharose, xylose, fructose |
| negative | arabinose, meso-inositol, mannose, rhamnose, raffinose, cellulose |

Characteristics

nitrate reduction: negative
starch hydrolysis: positive
tyrosine degradation: positive
milk peptonisation: positive
melanin formation: negative
growth temperature: 24-45° C
optimum pH: 7

As with any microorganism, Micromonospora sp. 87-16806 can be mutated or modified into different forms by conventional technique, e.g., by UV irradiation or by treatment with a mutagen such as N-methyl-N′-nitro-nitrosoguanidine. It is intended that all such mutant or modified forms, capable of producing dehydrosinefungin in a quantity greater than 10 mg/l of culture be within the scope of this application.

**Example 1**

Cultivation of Micromonospora sp. A87-16806

A) Starting culture on agar

An agar slant of Micromonospora sp. A87-16806 (Budapest Treaty deposit No. DSM 4757) is cultured for 14 days at 27° C in the following agar medium:

| | |
|---|---|
| glucose | 10 g |
| soluble starch | 20 g |
| yeast extract (Gistex®) | 5 g |
| casein hydrolysates* | 5 g |
| calcium carbonate | 1 g |
| agar (Bacto®) | 15 g |
| demineralized water (to a volume of 1 liter) | |
| pH neutralized to 7 with NaOH | |
| sterilization: 20 minutes at 120° C. | |

* Pancreatic hydrolysate of casein (N-Z Amine A Sheffield Chemical Co.)

B) Preculture

The spores and mycelium from 6 starting cultures are suspended in a 0.9% NaCl solution. A 2 liter Erlenmeyer flask containing 1 liter of preculture medium is inoculated with 20 ml of this suspension.

| Composition of the preculture medium | |
|---|---|
| glucose | 7g |
| soluble starch | 10g |
| malt extract (Wander®) | 5g |
| yeast extract (BBL®) | 2.7g |
| casein hydrolysates | 2.5g |
| calcium carbonate | 0.05g |
| sodium chloride | 0.03g |
| Iron II sulfate | 5mg |
| zinc sulfate | 4mg |
| Manganese II chloride | 2mg |
| Cobalt II chloride | 2mg |
| copper II sulfate | 0.2mg |
| boric acid | 0.1mg |
| potassium iodide | 0.05mg |
| demineralized water (to a volume of 1 liter) | |

The pH of the preculture medium is adjusted to 7 with NaOH/$H_2SO_4$ prior to sterilization at 120° C for 20 minutes.

The incubation of this preculture follows on a rotary shaking machine having an excentricity of 50 mm at 200 rpm for 96 hours at 27° C.

C) Intermediate culture

Three 12 liter Newbrunswick glass fermenters each containing 10 liters of preculture medium are inoculated with 1 liter of preculture and incubated at 27° C for 72 hours. The fermenters are rotated at 100 rpm during the first 24 hours and thereafter at 150 rpm. Air is introduced at a rate of 0.2 liter per minute per liter of medium during the first 24 hours and thereafter at 0.6 liters per minute per liter of medium.

D) Main culture

Three 125 liter fermentation vessels each containing 100 liters of main culture medium are inoculated with 10 liter of intermediate culture.

| Composition of main culture | |
|---|---|
| soluble starch | 20g |
| glucose | 5g |
| yeast extract (Difco) | 1.5g |
| casein hydrolysates | 1.5g |
| ammonium succinate | 3.1g |
| $K_2HPO_4$ | 0.4g |
| $KH_2PO_4$ | 0.2g |
| $MgSO_4 \cdot 7H_2O$ | 0.2g |
| $CaCl_2 \cdot 6H_2O$ | 0.05g |
| NaCl | 0.05g |
| $Fe(II)SO_4 \cdot 7H_2O$ | 5mg |
| $ZnSO_4 \cdot 7H_2O$ | 4mg |
| $Mn(II)Cl_2 \cdot 4H_2O$ | 2mg |
| $CoCl_2 \cdot 6H_2O$ | 2mg |
| $CuSO_4 \cdot 5H_2O$ | 0.2mg |
| $H_3BO_3$ | 0.1mg |
| KJ | 0.05mg |
| demineralized water (to a volume of 1 liter) | |

The pH of the main culture medium is adjusted to 6.5 with KOH/HCl. The main culture medium is then sterilized at 120° C for 20 minutes.

The main culture is incubated for 6 days at 27° C. The fermentation vessels are rotated at a rate of 80 rpm during the first 24 hours and thereafter at 100 rpm. Air is introduced at a rate of 0.5 liter per minutes per liter of medium during the first 24 hours and thereafter at 0.8 liter per minute per liter of medium. The pressure is 0.5 bar.

Isolation of Dehydrosinefungin from main culture:

The progress of the fermentation and the level of dehydrosinefungin in the culture medium can be followed during the growth period by analytical high performance liquid chromatography (HPLC). It can also be followed by testing samples of the culture medium for their antibiotic or herbicidal activity against organisms known to be sensitive to the compound, e.g., Aspergillus niger . The bioassay can be carried out by standard assay procedures, for example, the paper disc assay method. Also the aquatic plant Lemna minor , which is very sensitive to dehydrosinefungin, can be used to detect the progress of the fermentation. In general, maximum production of dehydrosinefungin occurs within about two to six days when submerged aerobic fermentation is carried out in large tanks or flasks.

Dehydrosinefungin can be recovered from the fermentation medium by employing extractive or adsorptive techniques. Adsorptive techniques are preferable to extraction procedures because the latter generally require the use of large volumes of solvents. Cationic exchange resins, such as the resins of the

polystyrenesulfonic acid and methacrylic carboxylic acid type, for example IRC-50® can be used as adsorbents for the recovery and isolation of dehydrosinefungin. The fermentation is first filtered to remove the mycelium. The use of a filter aid such as Celite 545® is desirable. The filtered broth is then passed over a cationic exchange resin, preferably IRC-50® in the ammonium cycle. The resin containing the adsorbed pesticides is washed with water to remove undesired impurities and the pesticides are then eluted from the resin with ammonium hydroxide at a normality of 0.5 N to 2.0 N. The eluate fractions containing dehydrosinefungin are combined and ammonia is evaporated. The eluate is concentrated in vacuo to a low volume. Upon the addition of a water soluble organic solvent, such as methanol, ethanol or acetone, dehydrosinefungin can be precipitated as a white solid and is collected by filtration.

The crude dehydrosinefungin preparation thus obtained can be further purified; for some purposes it may, however, be desirable for economic reasons to use it without further purification. The compound can be further purified by chromatography over non-ionic adsorbent materials, such as activated carbon, cellulose, dextran, silica gel, alumina and reversed phase C8®, C18®. Dehydrosinefungin is preferably obtained in a substantially pure state by successive chromatography over carbon or altenately over IRC-50® ($NH_4^+$) followed by chromatography over reversed phase C18. A preferred adsorbent is IRC-50® ($NR_4^+$) and the chromatography is carried out by stepwise gradient elution technique. Dehydrosinefungin is separable from sinefungin by eluting the cation exchange resin with ammonium hydroxide having a normality of between 0.05 N and 0.5 N. In the final purification step, the so obtained pesticide is further chromatographed over a C18 reversed phase column. The compound can be eluted from the column with 0.01-0.05% trifluoro acetic acid. After lyophylisation substantially pure dehydrosinefungin can be obtained in the late eluate fractions.

## EXAMPLE 2

The broth from three 100 l fermentations carried out as described in Example 1 are combined and filtered with the aid of Westfalia-Separator SB 7. The 300 l of filtrate so obtained are adjusted to pH 5 with diluted sulfuric acid, and are applied to a chromatography column containing 60 l of Amberlite IRC-50® ($NH_4^+$) resin. The column is washed with 120 l of deionized water and then eluted with a stepwise gradient of 0.5 N (Fraction I, total 60 l), 1N (Fraction II, total 60 l) and 2N (Fraction III, total 60 l) ammonium hydroxide and the elution is monitored by HPLC.

**Retention time of analytical HPLC:**

a. Column: Whatman Partisphere 5, SCX® column (110 x 4.7 mm)
Particle size: 5 um
Detection: UV 254 nm
Solvent system; 0.1M $NaH_2PO_4$, pH 4.52
flow rate; 1.4 ml/minute
Using this system, dehydrosinefungin has the following approximate retention time: 2.44 minutes

b. Column: Hewlett Packard ODS-C18 Hypersil® column (100 x 2.1 mm)
Particle size: 5 um
Detection: UV 254 nm
Solvent system: Pump A = acetonitril
Pump B = 0.05 % trifluoroacetic acid in water linear gradient 0 % A (= 100 % B) to 60 % A in 7 minutes.
Flow rate: 0.7 ml/minute

Using this system, dehydrosinefungin has the following approximate rentention time: 1.82 minutes.

Fraction II is rich in dehydrosinefungin and is applied further to a chromatography column containing 2 l of Amberlite IRC-50 ($NH_4^+$) resin. The column is washed with 10 l of water and is then eluted with a continuous gradient from water to 0.5 N ammonium hydroxide. Multiple 200 ml fractions are collected and the elution is monitored by HPLC. Fractions 21-27 are rich in dehydrosinefungin and are combined and concentrated in vacuo to approximately 50 ml and lyophillozed. The so obtained powder from fractions 21-27 (14 g) is dissolved in 100 ml water and applied to a chromatography column containing 2 l of reversed phase C-18 (size 40-634 um) resin. The column is washed with 2 l of water and then eluted with a continuous gradient from water to 0.02 % trifluoro acetic acid in water. Multiple 100 ml-fractions are collected and the elution is monitored by HPLC. Fractions 44-53 contain dehydrosinefungin and are combined, evaporated under reduced pressure and lyophillized to yield 3.56 g (11.9 mg/l of culture) of pure dehydrosinefungin.

## Claims

1. A microorganism capable of producing dehydrosinefungin in a culture in a quantity greater than 10 mg/l of culture.

2. The microorganism of claim 1 comprising a member of the genus Micromonospora .

3. The microorganism of claim 2 comprising Micromonospora sp . A87-16806 (DSM 4757) and mutant or modified forms thereof.

4. A method of producing dehydrosinefungin which comprises cultivating the microorganism defined in claim 1 under aerobic conditions in a culture medium containing assimilable sources of carbon, nitrogen and mineral salts to produce and accumulate dehydrosinefungin in said culture medium.

5. The method of claim 4 wherein the microorganism is a member of the genus Micromonospora .

6. The method of claim 5 wherein the microorganism is Micromonospora sp. A 87-16806 (DSM 4757) or a mutant or modified forms thereof.

7. A method of combatting weeds comprising applying to the weeds or their locus a herbicidally effective amount of dehydrosinefungin.

8. A method of combatting acari comprising applying to the acari or their locus an acaricidally effective amount of dehydrosinefungin.

9. A method of combatting plant pathogens comprising applying to the plant pathogens or their habitat a fungicidally effective amount of dehydrosinefungin.

10. An agricultural composition comprising a herbicidally, acaricidally, or fungicidally effective amount of dehyrosinefungin and an agriculturally acceptable carrier.